# FASCICULE DE BREVET EUROPEEN

(11) **EP 2 018 155 B1**
(45) Date de publication et mention de la délivrance du brevet: **28.12.2016**
(21) Numéro de dépôt: 07731392.2
(22) Date de dépôt: 30.04.2007
(51) Int. Cl.: A61K 8/02, A61K 8/14, A61K 8/73, A61Q 19/00, A61K 9/127, A61K 47/40

(54) **PROCEDE DE CONCENTRATION EXTEMPORANEE ET REVERSIBLE DE LIPOSOMES**
VERFAHREN ZUR REVERSIBLEN KONZENTRATION VON LIPOSOMEN
METHOD FOR REVERSIBLE CONCENTRATION OF LIPOSOMES

(30) Priorité: 03.05.2006 FR 0603945
(43) Date de publication de la demande: 28.01.2009
(73) Titulaire: Centre National de la Recherche Scientifique (C.N.R.S.), 75016 Paris (FR)
(72) Inventeur: LESIEUR, NÉE BOIVIN, Sylviane, F-92330 Sceaux (FR); BERNAT, Valérie, F-92290 Chatenay Malabry (FR); LE BAS, Geneviève, F-75014 Paris (FR); RINGARD, NÉE LEFEBVRE, Catherine, F-92500 Rueil Malmaison (FR)
(74) Mandataire: Lavoix
(86) Numéro de dépôt international: PCT/FR2007/000742
(87) Numéro de publication internationale: WO 2007/125217

(56) Documents cités:
- NISHIJO J ET AL: "INTERACTIONS OF CYCLODEXTRINS WITH DPPC LIPOSOMES. DIFFERENTIAL SCANNING CALORIMETRY STUDIES" CHEMICAL AND PHARMACEUTICAL BULLETIN, PHARMACEUTICAL SOCIETY OF JAPAN, TOKYO, JP, vol. 46, no. 1, janvier 1998 (1998-01), pages 120-124, XP000738277 ISSN: 0009-2363
- RICHARD L. MAGIN, HSIAO-CHANG CHAN: "Rapid separation of liposomes using ultrafiltration" BIOTHECHNOLOGY TECHNIQUES, [Online] vol. 1, no. 3, 1987, pages 185-188, XP009077376 Extrait de l'Internet: URL:http://www.springerlink.com/content/q7 q04819h8p41174> [extrait le 2007-01-17]

## Description

La présente invention concerne une série de procédés de préparation d'agrégats mixtes liposomes-cyclodextrines, et de liposomes, tels que revendiqués, les agrégats mixtes liposomes-cyclodextrines susceptibles d'être obtenus selon le procédé de la revendication 1, et leurs utilisations dans le domaine pharmaceutique, diagnostique ou cosmétique.

Les liposomes sont des vésicules (quelques dizaines de nanomètres à plusieurs microns) délimitées par une ou plusieurs bicouches de (phospho)lipides enfermant un volume aqueux. Ces structures résultent ainsi de l'arrangement de molécules amphiphiles généralement dispersées dans un large excès d'une phase aqueuse continue. Les parties hydrophiles ou têtes polaires des (phospho)lipides sont orientées de part et d'autre des feuillets bimoléculaires constituant les bicouches, toujours en contact avec le continuum aqueux, tandis que leurs chaînes forment le coeur des membranes, minimisant leur contact avec l'eau. Cette structure particulière, faite de compartiments aqueux séparés par des membranes relativement hydrophobes, confère aux liposomes, outre leur biocompatibilité, la propriété essentielle de pouvoir admettre des composés hydrosolubles ou lipophiles et de les transporter. Dans les domaines pharmaceutique et cosmétique, les liposomes, qu'ils soient formés de lipides naturels ou synthétiques, deviennent alors des agents potentiels pour la vectorisation de substances actives vers le tissu cible. De façon plus large, les liposomes sont des nano- ou micro-réservoirs pouvant contenir et véhiculer tous les types de produits. Leurs applications effectives et potentielles touchent également d'autres secteurs allant de la biologie fondamentale jusqu'à l'industrie agro-alimentaire ou agrochimique (modèle cellulaire, transfert de gènes, réacteurs chimiques ou enzymatiques, formulation de pesticides...).

Les méthodes existantes permettant de concentrer des liposomes, telles que l'ultracentrifugation ou l'ultrafiltration, sont lourdes à mettre en oeuvre, coûteuses, difficilement transposables à l'échelle industrielle. De plus, tout processus de floculation et de sédimentation de liposomes, qu'il soit spontané ou induit par apport d'une substance exogène ou d'énergie, conduit le plus souvent à l'agrégation irréversible des liposomes avec le risque d'engendrer leur fusion, ce qui entraîne inévitablement la perte des caractéristiques individuelles des liposomes (dimensions, composition, modification du contenu).

Les liposomes peuvent être également concentrés par lyophilisation. Cependant, le plus souvent, lorsque le lyophilisat est réhydraté, il y a modification des caractéristiques initiales des liposomes.

Les cyclodextrines sont des oligosaccharides cycliques composés d'unités glucopyranose liées entre elles par une liaison oside alpha (1-4), issus de l'amidon. Leur structure macrocyclique chirale prend la forme d'un cône tronqué qui délimite une cavité interne très hydrophobe (squelettes hydrocarbonés des unités glucose) alors que la partie externe reste hydrophile (groupements hydroxyle des unités glucose). Il existe une grande variété de cyclodextrines naturelles ou modifiées qui font l'objet de nombreuses études et applications dans les domaines de la recherche fondamentale, de l'industrie pharmaceutique, cosmétique, agro-alimentaire ou agrochimique. Ceci est dû à la capacité des cyclodextrines à former des complexes d'inclusion avec des molécules hydrophobes en permettant ainsi leur dispersion moléculaire en milieu solvant (aqueux) tout en les protégeant d'éventuelles dégradations. Par ailleurs, elles ont démontré d'excellentes propriétés en tant qu'excipients de compression de produits solides, émulsionnants ou promoteurs d'absorption.

Plusieurs systèmes particulaires contenant des cyclodextrines sont connus. Il s'agit notamment de nanosphères de poly(cyanoacrylate) formées en présence de cyclodextrines [polymérisation anionique en émulsion], de nanocapsules à contenu huileux ou de nanoparticules matricielles composées de cyclodextrines amphiphiles [procédés de nanoprécipitation, émulsion-évaporation de solvant, élimination de tensioactif de micelles mixtes], de microsphères d'éthylcellulose encapsulant des complexes cyclodextrines-principe actif hydrophobe [procédé émulsion-évaporation de solvant], de microcapsules [préparées par réticulation interfaciale de la béta-cyclodextrine avec du chlorure de téréphthaloyl], de billes [simple mélange d'alpha-cyclodextrine et de triglycérides].

Des systèmes mixtes liposomes-cyclodextrines ont également été décrits. Ces systèmes se présentent sous la forme de liposomes encapsulant dans leur compartiment aqueux un complexe cyclodextrine-principe actif. Ils sont préparés en fabriquant les liposomes en présence du complexe cyclodextrine-principe actif. Les liposomes sont produits en large excès d'eau et en aucun cas l'obtention d'agrégats mixtes [liposomes-cyclodextrines] concentrés n'est décrite.

Il a maintenant été mis au point un procédé de concentration extemporané et réversible de liposomes permettant la conservation des caractéristiques initiales des liposomes.

Plus spécifiquement, de façon inattendue, les inventeurs ont mis en évidence que l'addition de cyclodextrines à des liposomes en dispersion aqueuse permet de provoquer la floculation puis la sédimentation des liposomes, ce par quoi une concentration de liposomes sous forme d'agrégats mixtes liposomes-cyclodextrines est obtenue. Ces agrégats mixtes sont stables en l'état, allient les propriétés individuelles des liposomes et des cyclodextrines et sont en outre aisément lyophilisables.

Avantageusement, le lyophilisat peut être hydraté sans que la structure initiale des liposomes ne soit affectée.

Le procédé selon l'invention présente de nombreux autres avantages.

En particulier, les liposomes peuvent contenir des substances hôtes sans que ces dernières ne soient perdues lors du procédé. Ils peuvent par ailleurs être redispersés par simple dilution (système réversible).

De manière avantageuse, ce procédé d'agrégation et de desagrégation ne fait appel ni à l'utilisation de solvants organiques, ni à une étape de chauffage, ni à une consommation importante d'énergie. De plus, il ne modifie pas les caractéristiques physico-chimiques des entités de départ, ce qui constitue une avancée de grand intérêt dans le domaine de la concentration de liposomes.

En outre, ce procédé de concentration de liposomes ne nécessite pas d'appareillages spéciaux tels que des centrifugeuses. Aucune agitation n'est requise. Le procédé de fabrication ne fait pas intervenir de solvants organiques ni de chauffage, ce qui présente un avantage en terme de sécurité.

Enfin, les matériaux utilisés sont facilement disponibles sur le marché et à un coût modéré. Le procédé peut être aisément transposé à grande échelle.

L'invention fournit ainsi des moyens de grande simplicité et de faible coût pour concentrer et redisperser des liposomes utilisables dans de nombreux secteurs de l'industrie.

De plus, les agrégats mixtes liposomes-cyclodextrines obtenus par concentration des liposomes en présence de cyclodextrines sont généralement de taille micronique et peuvent avantageusement trouver des applications nouvelles en cumulant les propriétés des liposomes et des cyclodextrines.

Selon un premier aspect, l'invention décrit un procédé de concentration de liposomes comprenant une étape de formation d'agrégats mixtes liposomes-cyclodextrines par mise en contact de liposomes dispersés en milieu aqueux avec des cyclodextrines.

Par « cyclodextrine », on entend des oligosaccharides cycliques composés d'un enchaînement en α-1,4 d'au moins six unités D-glucopyranose, hydrosolubles, issus de l'amidon, possédant une cavité hydrophobe capable de piéger des molécules lipophiles. Les cyclodextrines au sens de la présente description englobent les cyclodextrines naturelles ou leurs dérivés sous réserve toutefois que ces derniers soient hydrosolubles, ne présentent aucune propriété de détergence ou solubilisante et ne développent pas d'interactions spécifiques avec les lipides constituants des liposomes qui pourraient engendrer la perte de la structure vésiculaire de ceux-ci lors de l'utilisation du procédé.

Les cyclodextrines naturelles sont des oligosaccharides cycliques obtenus par hydrolyse enzymatique de l'amidon. Elles comprennent les cyclodextrines α, β et γ (respectivement comprenant 6, 7 ou 8 unités glucose), les cyclodextrines-α étant particulièrement préférées.

Les dérivés de cyclodextrines désignent, au sens de la présente description, des cyclodextrines naturelles dont la structure native a été modifiée par liaison covalente d'un ou de plusieurs groupes chimiques, notamment hydrophiles. Il peut s'agir, par exemple, d'une cyclodextrine naturelle dont l'une au moins des fonctions hydroxyle est substituée par un groupe saccharidique. Les dérivés de cyclodextrines au sens de la présente description comprennent également des cyclodextrines polymérisées.

Tout ou partie des cyclodextrines utiles selon le procédé de l'invention peuvent être mises en oeuvre sous forme de complexe d'inclusion, *i.e.* contenir une molécule hydrophobe de petite taille, désignée ci-après "molécule hôte". Des exemples de molécules hôtes sont notamment des molécules pouvant développer des propriétés spectroscopiques (tels que les colorants ou les pigments), des radio-éléments (tels que l'iode), des principes actifs thérapeutiques (tels que certaines vitamines, anti-inflammatoires).

Par "liposome", on entend une vésicule dont la paroi est formée d'une ou plusieurs bicouches de molécules amphiphiles renfermant un espace interne aqueux, lesdites molécules amphiphiles comprenant une tête polaire et des résidus hydrophobes, le plus souvent des chaînes alkyle ou "queues hydrophobes".

De préférence, la ou les bicouche(s) des liposomes comprennent des phospholipides.

De façon particulière, le terme "liposome" peut désigner une cellule biologique, procaryote ou eucaryote, animale ou végétale, ou une membrane biologique reconstituée de type « ghost » ("fantôme").

Comme exemple de phospholipides, on peut citer la phosphatidylcholine (PC) et ses dérivés : l'egg phosphatidylcholine (Egg-PC), la dimyristoyl-phosphatidylcholine (DMPC), la dipalmitoylphosphatidylcholine (DPPC), la dioléoylphosphatidylcholine (DOPC), la dilauroylphosphatidylcholine (DLPC), la distéaroylphosphatidylcholine (DSPC), la diarachidoylphosphatidylcholine (DAPC) et la dilinoléoylphosphatidylcholine (DLPC).

D'autres phospholipides, comprenant par exemple un groupement glycérol lié à deux chaînes d'acides gras et dont la tête polaire est différente de la phosphatidylcholine, peuvent être utilisés selon le procédé de l'invention.

D'autres molécules amphiphiles peuvent entrer en partie dans la composition des bicouches des liposomes (cholestérol, lipides dont la tête polaire est modifiée par un groupement hydrophile, lipides cationiques, lipides fluorescents...). De préférence, les liposomes comprennent majoritairement des phospholipides.

Il va sans dire que les liposomes selon la présente invention ne comprennent pas de molécules amphiphiles modifiées par greffage de polymères hydrophiles, comme celles décrites dans WO 2005/0300170, dont l'interaction avec des cyclodextrines conduit à la déstabilisation du liposome.

Les liposomes peuvent être préparés selon des techniques conventionnelles parmi lesquelles on peut citer l'irradiation par ultrasons, l'inversion de phase, l'extrusion, la dialyse, l'absorption sur résine ou la filtration sur gel de micelles mixtes lipide-détergent, la congélation-décongélation. A titre d'exemple, les liposomes peuvent être préparés par hydratation d'un film de phospholipides suivie d'une extrusion séquencée pour calibrer les vésicules.

Le pH et la force ionique du milieu aqueux ne sont pas critiques. Ainsi, le milieu aqueux peut être de l'eau pure ou bien un milieu aqueux tamponné, notamment à un pH compris entre 6 et 8, ou bien un milieu aqueux contenant un sel monovalent, notamment un cation et un anion monovalents, comme le chlorure de sodium. Ce dernier peut être ajouté à une concentration préférée inférieure ou égale à 150 mM. Des exemples de tampons utiles selon l'invention sont notamment le tampon phosphate, le tampon PBS, le tampon Hepes, le tampon Hepes + NaCl. Les tampons permettent avantageusement de prolonger la stabilité dans le temps des agrégats mixtes liposomes-cyclodextrines concentrés obtenus selon le procédé.

La concentration molaire des liposomes dans le milieu aqueux n'est pas critique et peut varier dans une large mesure. De préférence, elle est supérieure à 0,05 mM, et est plus préférentiellement comprise entre 0,5 mM et 5 mM.

Il y a lieu de préciser que la concentration molaire de liposomes se réfère ici à la concentration molaire des molécules amphiphiles tels que les phospholipides constituant la ou les bicouche(s) de la paroi du liposome.

De même, la concentration des cyclodextrines dans le milieu aqueux n'est pas critique et peut varier entre 10 mM et 80 mM, notamment entre 20 mM et 40 mM, notamment pour des liposomes de dioléoylphosphatidylcholine.

De préférence, le rapport des concentrations molaires cyclodextrines/liposomes dans le milieu aqueux est supérieur à 1, en particulier compris entre 2 et 1500.

En général, il a été observé que plus le rapport molaire cyclodextrine-liposome est élevé, plus l'agrégation des liposomes via les phénomènes de floculation et de sédimentation est rapide. De même, plus la concentration des liposomes et/ou des cyclodextrines est élevée, plus le processus de sédimentation est rapide.

De préférence, on additionne les cyclodextrines sur les liposomes dispersés en milieu aqueux. Cette addition est préférentiellement contrôlée en concentration et/ou en vitesse d'addition (instantanée ou progressive).

Selon un mode de réalisation de l'invention, tout ou partie des liposomes contiennent une substance exogène, hydrophile ou hydrophobe. Il peut s'agir notamment d'un principe actif cosmétique, thérapeutique, diagnostique, ou encore des arômes, des parfums, des nutriments, des vitamines, des pesticides.

Ces substances sont généralement des molécules ou des macromolécules dont le passage au travers de la bicouche du liposome est très limité, voire nul.

Les agrégats mixtes obtenus selon le procédé peuvent être récupérés selon des techniques conventionnelles telles que la filtration ou la centrifugation sans qu'ils soient détruits.

Selon un mode de réalisation particulièrement préféré de l'invention, le procédé comprend de plus une étape de lyophilisation des agrégats mixtes liposomes-cyclodextrines obtenus selon le procédé.

Selon un autre mode de réalisation préféré de l'invention, le procédé comprend de plus une étape de dialyse des agrégats mixtes liposomes-cyclodextrines obtenus selon le procédé.

Selon un autre mode de réalisation préféré de l'invention, le procédé comprend de plus une étape de réhydratation du lyophilisat des agrégats mixtes liposomes-cyclodextrines suivie d'une étape de dialyse.

Selon une variante, tout ou partie des liposomes représente(nt) des cellules biologiques. A titre d'exemple, le milieu aqueux peut contenir un mélange de liposomes encapsulant éventuellement une substance active et des cellules biologiques. Le procédé selon l'invention comprend alors une étape de formation d'agrégats mixtes liposomes-cellules biologiques-cyclodextrines.

Le procédé selon l'invention est, selon cette variante, particulièrement utile pour favoriser les interactions entre les liposomes et les cellules biologiques, notamment leur fusion ou leur internalisation ou encore les interactions entre cellules biologiques. Le procédé peut ainsi permettre d'améliorer le passage d'une substance active contenue dans les liposomes vers l'intérieur de la cellule biologique.

Selon un deuxième aspect, l'invention a pour objet des agrégats mixtes de liposomes et de cyclodextrines susceptibles d'être obtenus par le procédé selon la revendication 1.

De manière avantageuse, ces agrégats mixtes associent les propriétés des cyclodextrines, d'une part, et des liposomes, d'autre part. Ils peuvent ainsi encapsuler des molécules hydrophobes dans la partie cyclodextrine et/ou des molécules ou macromolécules hydrophobes ou hydrophiles dans la partie liposome. Ils sont, en outre, avantageusement dissociables par dilution, sans que les propriétés individuelles des composants ne soient affectées.

Ces agrégats mixtes peuvent se présenter sous la forme de particules, et possèdent généralement une taille micrométrique, notamment comprise entre 100 nm et 10 µm.

Selon une variante, les agrégats mixtes susceptibles d'être obtenus selon le procédé sont des agrégats mixtes liposomes-cellules biologiques-cyclodextrines.

Selon un autre aspect, l'invention concerne un mélange concentré de liposomes enrichi en cyclodextrines, notamment sous forme de lyophilisat, susceptible d'être obtenu selon le procédé de l'invention.

Selon un autre aspect, l'invention concerne l'utilisation des agrégats mixtes liposomes-cyclodextrines susceptibles d'être obtenus selon le procédé de l'invention pour encapsuler des substances exogènes, hydrophiles ou hydrophobes, en particulier dans des compositions cosmétiques, pharmaceutiques ou diagnostiques.

L'invention est en effet particulièrement utile pour concentrer des liposomes destinés à être utilisés comme transporteurs ou vecteurs de principes actifs thérapeutiques ou diagnostiques, en pharmacie ou en cosmétique. Par exemple, les agrégats mixtes liposomes-cyclodextrines peuvent être utilisés pour une application topique par voie transdermique. En effet, des associations binaires ou tertiaires de promoteurs d'absorption montrent des effets synergiques liés à leur effets propres ; or, les phospholipides et l'α-cyclodextrine sont tous deux des promoteurs d'absorption utilisables dans le cadre de dispositifs transdermiques. Les agrégats mixtes obtenus par le procédé de l'invention peuvent également être utilisés pour l'administration par voie orale ou par absorption par les muqueuses.

L'invention décrit également, selon un autre aspect, l'utilisation de cyclodextrines à titre de précurseurs de sédimentation de liposomes en milieu aqueux.

### FIGURES

Figure 1 : Exemple de variation de la turbidité d'une dispersion de liposomes par ajout de cyclodextrine en fonction du temps. Le floculat est obtenu à partir d'un mélange de liposomes de DOPC (0.49 mM), de tampon Hepes (pH =7.4) et d'une solution d' α-CD (42.09 mM) à 25°C. La densité optique (DO) est mesurée à 400 nm.
Figure 2 : Etapes de concentration de liposomes après addition de cyclodextrines. Le système est ici composé de liposomes de DOPC (0.49 mM), de tampon Hepes (pH=7.4) et d'une solution d' α-CD (42.09 mM).

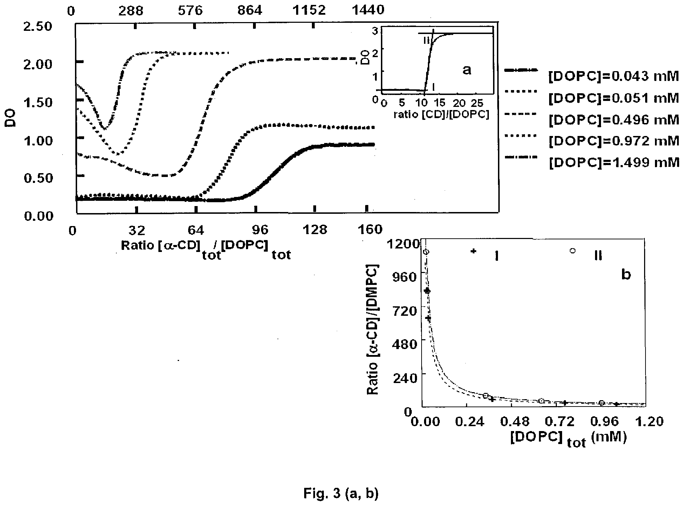

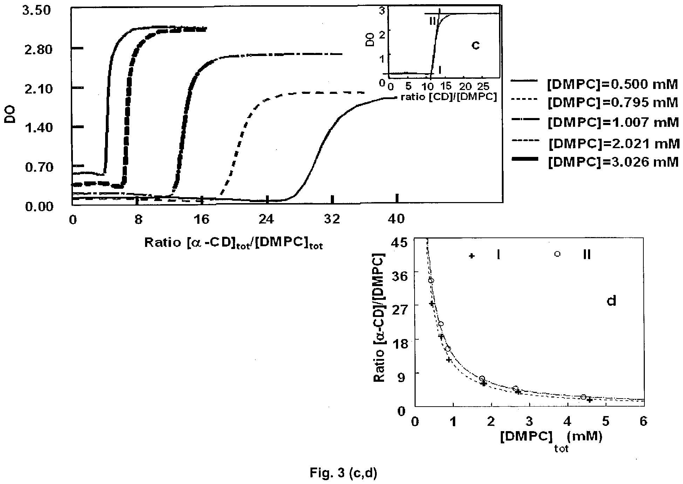

Figure 3 : Exemples d'étude du procédé de concentration de liposomes par mesure de turbidité (DO à 400 nm). Les courbes des figures a et c montrent l'augmentation de DO en fonction de la concentration en cyclodextrine pour des concentrations en liposomes initiales variables (concentrations en lipide initiales reportées en légende) pour des liposomes de DOPC (a) et de DMPC (c). Pour chaque courbe, le plateau obtenu correspond à la formation d'agrégats mixtes liposomes-cyclodextrines. Les courbes des figures b et d montrent la variation de la proportion cyclodextrine/lipide en fonction de la concentration en lipide pour obtenir le phénomène de floculation pour la DOPC (b) et la DMPC (d). Les systèmes sont ici composés de liposomes de DOPC (dioléoyl phosphatidylcholine) ou de DMPC (dimyristoyl phosphatidylcholine) et d'α-cyclodextrine. Les inset représentent la méthode de détermination des rapports cyclodextrine/lipide encadrant la remontée en DO précédant le plateau des courbes de turbidité.

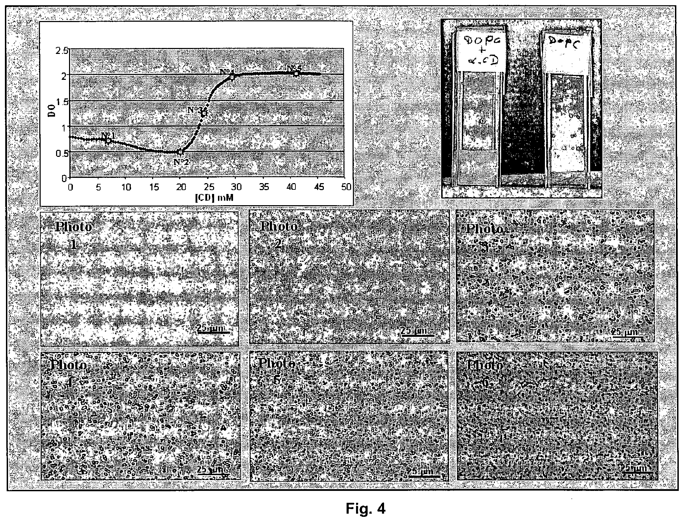

Figure 4 : Illustration du processus de concentration des liposomes de DOPC en présence d'α-CD. Etude effectuée par mesure de turbidité (DO à 400 nm) et par microscopie optique (X40). Chaque point reporté sur la courbe de turbidité correspond à un échantillon analysé en microscopie. La photographie de droite montre d'une part les liposomes avant ajout de cyclodextrine puis dans l'état final d'agrégation au plateau de la courbe de turbidité. Lorsque la DO augmente, les liposomes se concentrent et floculent, ce qui se traduit en microscopie optique par l'augmentation de la concentration des objets dans un volume donné de l'échantillon. L'image photo 1 montre les liposomes en l'absence de cyclodextrine: ils sont très dilués et trop petits pour être visualisés.
Figure 5 : Images de microscopie confocale de fluorescence de liposomes de DOPC marqués à la Rhod-PE (rhodamine-distéaroylphosphatidyl-éthanolamine) avant et pendant leur agrégation par ajout de cyclodextrine en fonction du temps.
Figure 6 : Intensité d'émission de fluorescence de la calcéine contenue dans des liposomes de DOPC avant et après agrégation. Les spectres ont été enregistrés après avoir solubilisé les liposomes par un détergent l'octyl glucoside de façon à libérer les molécules de calcéine piégées dans les liposomes. Les liposomes agrégés ont été préalablement séparés du continuum aqueux par centrifugation.
Figure 7 : Etude de la désagrégation de liposomes de DOPC préalablement agrégés par addition de cyclodextrine sous effet d'une simple dilution. Microscopie optique en champ noir (X40).
Figure 8 : Images de microscopie optique (X40) d'agrégats mixtes de liposomes de 800 nm de diamètre et de cyclodextrine.
Figure 9 : Images de microscopie optique (X40) et photographies des échantillons correspondants : de gauche à droite, liposomes sans cyclodextrine, lyophilisat d'agrégats mixtes liposome/cyclodextrine, liposomes après réhydratation du lyophilisat.

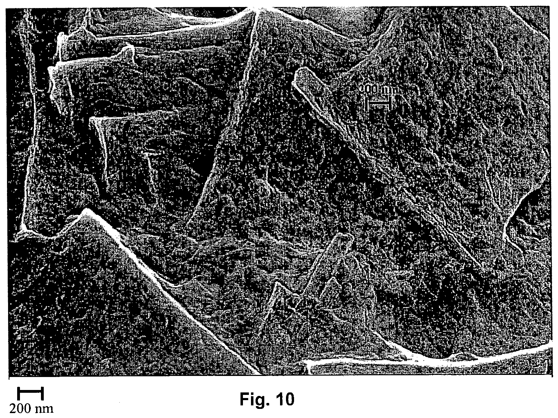

Figure 10 : Images par microscopie électronique à balayage de lyophilisat d'agrégats mixtes liposome/cyclodextrine. Les molécules de cyclodextrine forment une matrice découpée en plaques de section carrée de dimension voisine de 2 µm² dans lesquelles sont piégées des structures sphéroïdales de diamètre environ 200 nm, correspondant aux liposomes formant les agrégats précurseurs. Le procédé de lyophilisation conserve donc la structure des liposomes formant les agrégats mixtes liposome/cyclodextrine. L'appareil utilisé est un LEO 9530 (France). Les échantillons ont été recouverts d'un dépôt de Platine/Palladium avant analyse.
Figure 11 : Images de microscopie électronique par cryo-fracture de liposomes obtenus après dialyse des agrégats mixtes de liposomes de DMPC et d'α-cyclodextrine obtenus par le procédé de concentration (concentrations en DMPC : 0,4 mM et en α-cyclodextrine : 15,6 mM).
Figure 12 : Diamètres hydrodynamiques moyens (en nm) mesurés par diffusion quasi-élastique de la lumière de liposomes de DOPC ou de DMPC avant (a,c) et après (b,d) dialyse des agrégats mixtes de liposomes et d'α-cyclodextrine obtenus par le procédé de concentration. Les concentrations de phospholipides sont en DOPC : 0,3 mM (a,b) et en DMPC : 0,4 mM (c,d). La concentration en α-cyclodextrine utilisée pour le procédé de concentration est de 28,4 mM (b) ou 15,6 mM (d).

Les exemples suivants illustrent l'invention, sans toutefois la limiter. Les produits de départ utilisés sont des produits connus ou préparés selon des modes opératoires connus.

Les pourcentages sont exprimés en poids, sauf mention contraire.

### EXEMPLES

### Exemple 1 : Formation des agrégats (liposomes concentrés)

Le procédé s'applique à tout type de liposomes, notamment constitués majoritairement de phospholipides quels que soient leur mode de préparation et leur structure finale (taille, forme, composition). Typiquement, le procédé a été essayé sur des liposomes unilamellaires (diamètre moyen : 200 nm) et sur des liposomes oligolamellaires (diamètre moyen : 1 µm).

### Etape 1 : préliminaire

Dans un mode préféré de réalisation de la présente invention, les liposomes utilisés ont été préparés par hydratation d'un film de phospholipides suivie d'une extrusion séquencée pour calibrer les vésicules. Le film de phospholipides est obtenu par évaporation du solvant d'une solution chloroformique de phospholipide sous courant d'azote. Les traces de solvant résiduel sont éliminées par lyophilisation sous vide poussé pendant 12 heures. Ce film est ensuite hydraté par un volume déterminé d'une phase aqueuse (eau osmosée, tampon phosphate, tampon PBS, tampon Hepes, tampon Hepes + NaCl) de façon à ce que la concentration totale en phospholipides soit de 10 mM. La dispersion est maintenue au bain marie à 25°C et homogénéisée par agitation à l'aide d'un vortex. La dispersion finale est fluide et opalescente. Elle est ensuite extrudée par passages successifs sur des filtres de poly(carbonate) Poretics® (Osmonics, Livermore, USA) de diamètres de pores décroissants (0,8; 0,4 et 2×0,2 µm) à l'aide d'un extrudeur construit au laboratoire et fonctionnant sous pression d'azote. Après la dernière extrusion, des vésicules en majorité unilamellaires, de taille homogène et stables dans le temps, sont obtenues. L'évolution de la taille est suivie par diffusion quasi-élastique de la lumière (nanosizer Coulter Electronics LTD).

### Etape 2 : Procédé de concentration

### a) Floculation

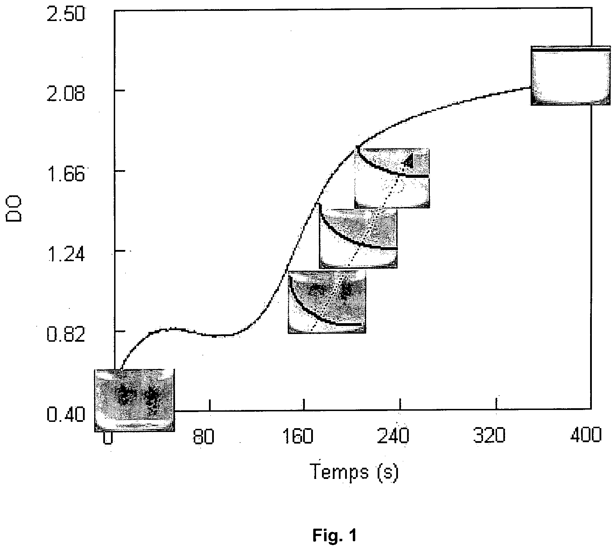

Les liposomes sont amenés à la concentration finale choisie par ajout de la phase aqueuse dispersante. On ajoute ensuite, en une seule fois, une solution d'α-cyclodextrine (α-CD) préparée dans le même milieu. Au bout d'une période comprise entre 1 et 10 minutes, les liposomes floculent sous la forme d'une dispersion d'agrégats de diamètres allant de 800 nm à quelques microns. La Figure 1 montre l'évolution de la turbidité de l'échantillon au cours du temps par mesure de la densité optique (DO). Plus la valeur de la DO est élevée, plus la proportion d'agrégats augmente. Le dernier plateau indique la période de temps nécessaire pour que le phénomène de floculation soit complet. Il a été observé que le temps estimé pour l'obtention des agrégats diminue avec l'augmentation de la concentration en α-cyclodextrine. Ainsi, la concentration de cyclodextrine est un paramètre régulateur du taux d'agrégation des liposomes dans la dispersion.

### b) Sédimentation

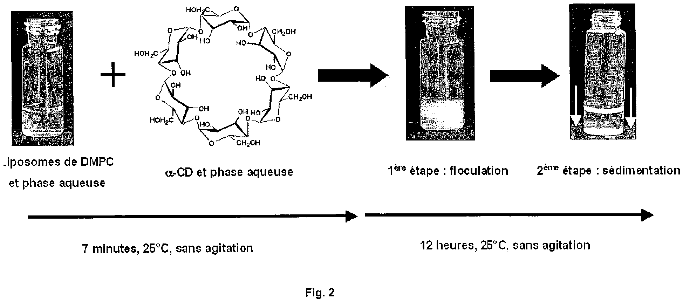

Après le phénomène de floculation, il se produit un processus de sédimentation spontanée entraînant par effet de densité le dépôt des agrégats mixtes liposomes-cyclodextrines (Figure 2). La durée de ce phénomène est comprise entre quelques heures et 24 heures selon les concentrations initiales des liposomes et de la cyclodextrine.

### Exemple 2 : Influence du rapport molaire cyclodextrines/phospholipides sur la concentration des liposomes

Le suivi de la turbidité des dispersions de liposomes par mesure de la densité optique (DO) au spectrophotomètre UV-Visible en fonction des proportions de cyclodextrine ajoutée, de phase aqueuse et de liposomes a permis de préciser la ou les zone(s) de formation des agrégats et donc de caractériser les conditions de concentration des liposomes.

Cet exemple a été réalisé sur des liposomes de DOPC unilamellaires obtenus par extrusion et de diamètre moyen 200 nm. Les liposomes de DOPC ont été placés à différentes concentrations en lipides (de 0,5 à 5 mM) dans une cuve en quartz permettant l'enregistrement en continu de la DO. Une solution d'α-cyclodextrine (80 mM) a été progressivement ajoutée. Les résultats obtenus (Figure 3 a,b) montrent que, pour obtenir la floculation des liposomes et donc leur concentration dans le milieu, plus la concentration en liposomes de départ est faible et plus la concentration en α-cyclodextrine ajoutée doit être élevée. La formation du floculat est visualisée par une augmentation brutale de la densité optique. Les proportions des trois constituants du mélange ont une incidence sur le temps de formation et la taille des agrégats obtenus.

**Tableau 1 : Influence du ratio [α-CD]/[DOPC] sur la formation du floculat (sa taille, son aspect microscopique (X40) et sa densité apparente (--) très faible, (-) faible, (+) moyenne, (++) forte, (+++) très forte. Voir aussi Figure 4**

| **[αCD] mM** | **[DOPC] mM** | **Ratio [αCD]/ [DOPC]** | **Densité du floculat** | **Taille des agrégats mixtes liposomes/ cyclodextrine** | **Situation sur la courbe** | **Microscopie optique (X40)** |
|---|---|---|---|---|---|---|
| 0 | 0.49 | 0 | -- | liposomes seuls : 0.2±0.017µm | Point n°1 | Photo 1 |
| 6.59 | 0.458 | 14.4 | - | 1,9± 0.2µm | Point n°2 | Photo 2 |
| 19.58 | 0.383 | 51.4 | + | 3 ± 1µm | Point n°3 | Photo 3 |
| 23.95 | 0.358 | 66.8 | ++ | 3.5± 1.5 µm | Point n°4 | Photo 4 |
| 28.4 | 0.333 | 85.3 | +++ | 4± 2 µm | Point n°5 | Photo 5 |
| 39.24 | 0.271 | 144.9 | +++ | 4.8± 3 µm | Point n°6 | Photo 6 |

La même expérience a été réalisée à partir de liposomes de dimyristoyl phosaptidylcholine DMPC (lipide synthétique à chaînes saturées). Les résultats sont illustrés par la Figure 3c,d.

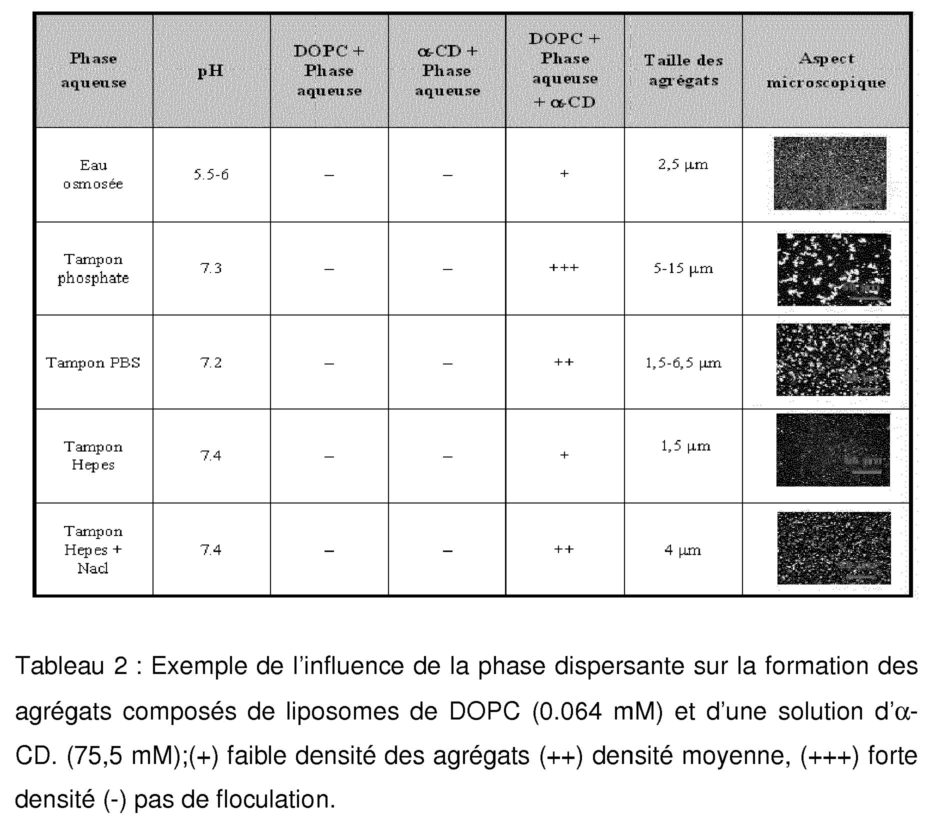

### Exemple 3 : Concentration de liposomes contenant une substance exogène Deux substances modèles ont été essayées.

### a) Exemple de molécule lipophile : la rhodamine-distearoylphosphatidyl-éthanolamine (Rhod-PE)

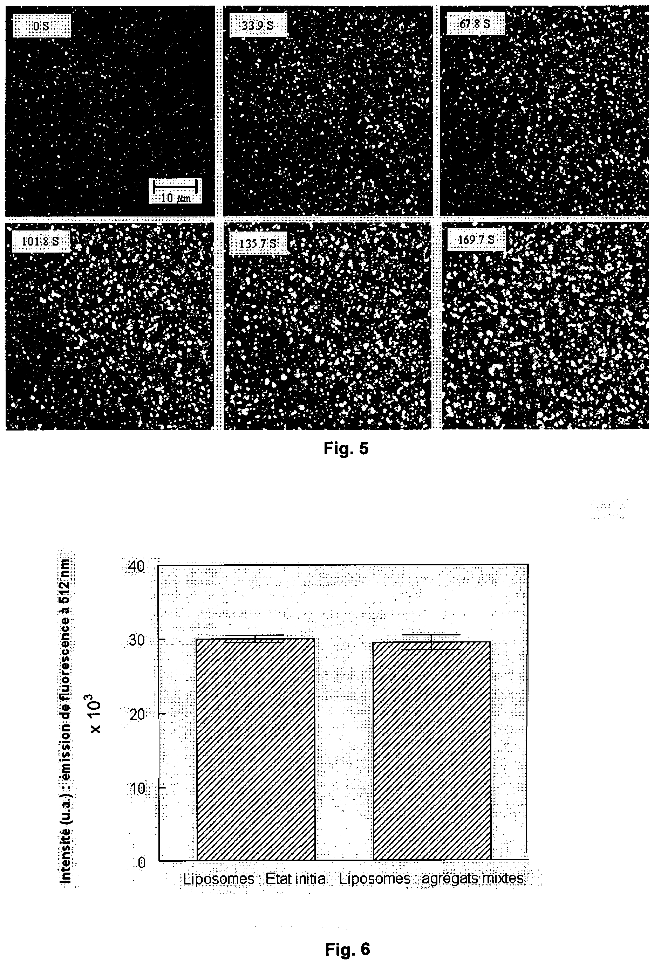

La rhod-PE est un phospholipide porteur d'un groupement fluorescent (rhodamine). L'étude a été réalisée par microscopie confocale de fluorescence. Les résultats montrent que le phénomène de floculation induit par l'α-cyclodextrine de liposomes chargés en Rhod-PE (préparés par la méthode décrite à l'exemple 1) ne modifie pas la fluorescence des liposomes. La microscopie confocale de fluorescence montre clairement des agrégats de liposomes fluorescents (Figure 5).

### b) Exemple de molécule hydrophile : la calcéine

La calcéine est une molécule fluorescente de faible masse molaire soluble dans l'eau. Aucune trace de calcéine préalablement encapsulée dans des liposomes de DOPC n'a été détectée par spectrophotométrie de fluorescence dans le surnageant après la sédimentation spontanée des liposomes induite par addition de cyclodextrine. De plus, la quantité de calcéine contenue dans les liposomes a été conservée dans les agrégats (Figure 6). Ainsi, l'imperméabilité de la membrane des liposomes ne semble pas être modifiée par les molécules de cyclodextrines.

### Exemple 4 : Réversibilité du procédé par simple dilution

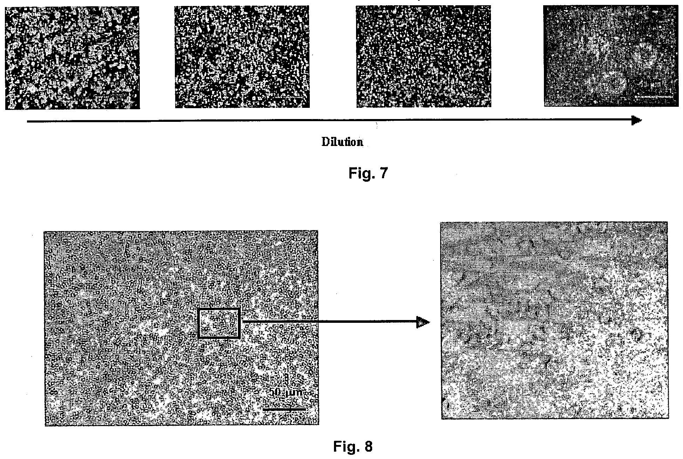

La première étape du procédé est effectuée comme décrit au paragraphe précédent. On obtient un concentré de liposomes et de cyclodextrines sous la forme d'un précipité blanc. Puis, on ajoute un volume de phase aqueuse (dilution finale × 100 en volume) et l'on suit l'évolution du système par microscopie optique (Figure 7). La désagrégation des liposomes se produit en quelques minutes, à mesure que la phase aqueuse progresse au sein du concentré de liposomes jusqu'à conduire à une dispersion de liposomes sans interactions dans le milieu ; ces derniers apparaissent alors comme de petits spots isolés par microscopie en champ noir.

La même expérience reproduite sur des liposomes contenant cette fois de la Rhod-PE a été réalisée. L'évolution du concentré de liposomes a été suivie par microscopie confocale de fluorescence (λexc=488 nm, λem= 543 nm) à 25°C. On observe nettement après dilution que les liposomes restent fluorescents et se redispersent dans le milieu.

La même expérience a également été réalisée avec des liposomes contenant de la calcéine dans leur volume aqueux interne. Les spectres d'émission de la calcéine enregistrés sur les préparations de liposomes avant agrégation puis après agrégation/redispersion sont superposables (forme et intensité). De plus, les diamètres moyens des liposomes mesurés par diffusion quasi-élastique de la lumière sont conservés.

### Exemple 5 : Caractérisation du mélange concentré liposomes-cyclodextrines (agrégats mixtes)

### Aspect macroscopique

Les agrégats formés par l'α-cyclodextrine et les liposomes forment une suspension qui sédimente dans le temps. La sédimentation est totale après un temps allant de quelques heures à 24 heures. Celle-ci permet la concentration des liposomes dans un très faible volume. Le système se redisperse par simple agitation, sans que les agrégats ne se dissocient. Les agrégats formés sont stables dans le temps, à température ambiante. Ils sont de couleur blanche et leur taille peut varier de 1 à 20 µm selon les concentrations en liposomes et cyclodextrines employées. Leurs dimensions peuvent évoluer sous l'action d'un faible cisaillement.

### Aspect microscopique

L'étude microscopique (X20) du système sur des liposomes de 800 nm de diamètre initial a montré que la présence de cyclodextrine entraîne l'agrégation spontanée des liposomes. Ces derniers forment ainsi des grappes de liposomes intacts (Figure 8).

### Aptitude à la lyophilisation

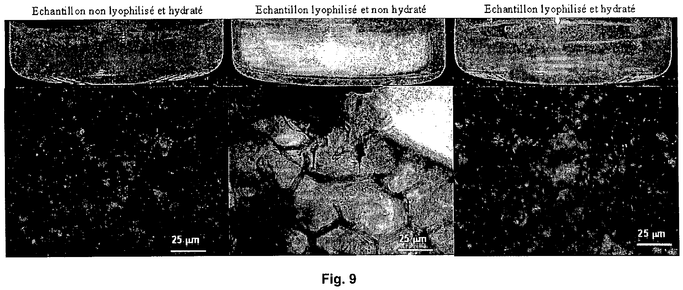

Il a été observé au microscope optique que les « grappes » de liposomes (DMPC (200 nm), α-cyclodextrine) ainsi formées peuvent subir une lyophilisation sans altérer les caractéristiques des liposomes pris individuellement. La réhydratation du lyophilisat permet de revenir à l'état de départ sans modifier l'aspect des liposomes (Figure 9).

L'échantillon produit par réhydratation du lyophilisat a été dilué de façon à dissocier les agrégats liposomes-cyclodextrines. Des mesures de diffusion quasi-élastique de la lumière ont montré que le diamètre initial des liposomes avant leur concentration (200 nm) était retrouvé après lyophilisation/réhydratation. Ainsi, le procédé de concentration mis au point est un excellent outil pour préparer des liposomes lyophilisés sans modifier leurs caractéristiques initiales. Des expériences de microscopie électronique à balayage ont été effectuées sur des échantillons mixtes liposome/cyclodextrine après agrégation et lyophilisation. Les images montrent nettement les liposomes, de dimensions très voisines de celles des liposomes au sein des agrégats hydratés, piégés dans une matrice de cyclodextrine (Figure 10).

### Exemple 6 : Dispersion des liposomes par dialyse des agrégats mixtes de liposomes et d'α-cyclodextrine.

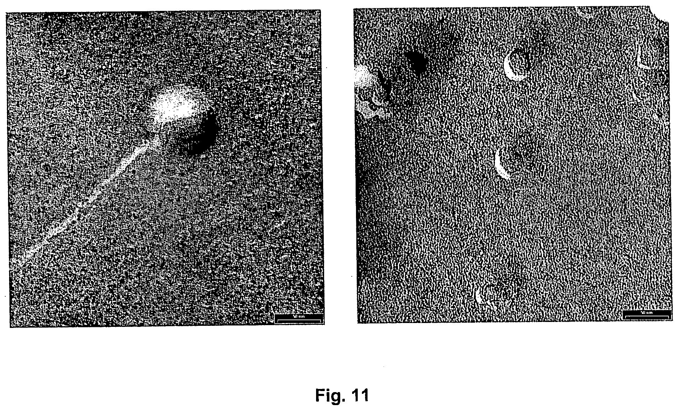

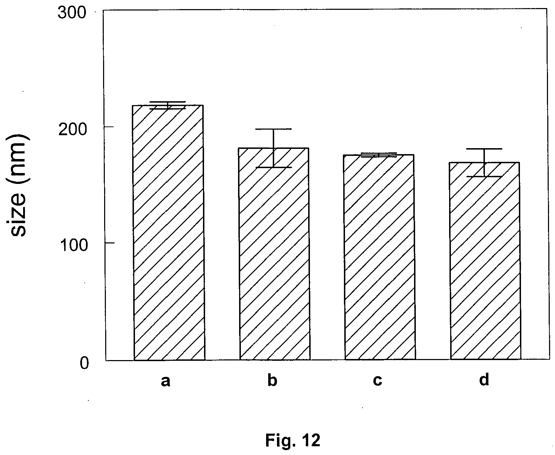

La première étape du procédé est effectuée comme décrit dans l'exemple 1. On obtient un concentré de liposomes et d'α-cyclodextrine sous la forme d'un précipité blanc. Typiquement, pour des concentrations initiales en phospholipides de 0,5 mM et d'α-cyclodextrine comprises entre 15 et 30 mM, on place 2 mL de la préparation contenant les agrégats mixtes de liposomes et d'α-cyclodextrine dans une chambre de dialyse munie d'une membrane Spectrapore (cutoff 12000 Da) laissant passer les molécules de cyclodextrine mais pas les liposomes. La chambre est ensuite placée dans un bécher de 1 L contenant 800 mL du milieu aqueux ayant été utilisé pour la fabrication des liposomes de départ. L'ensemble, chambre plus milieu aqueux, est agité doucement à l'aide d'un barreau aimanté à 25°C. Le bain de dialyse est renouvelé deux fois toutes les 24 heures. A la fin de la dialyse (2 x 800 mL par 24h pendant 3 jours), on récupère dans la chambre de dialyse une dispersion de liposomes individualisés (voir Figure 11) dans un volume égal à celui de l'échantillon de départ (2 mL). La concentration en liposomes après dialyse est celle au sein des agrégats mixtes obtenus par le procédé de concentration. L'élimination des molécules d'α-cyclodextrine a donc permis la désagrégation des liposomes dont le diamètre moyen mesuré par diffusion quasi-élastique de la lumière est très proche de celui des liposomes initiaux (voir Figure 12). Ceci montre comme dans l'exemple 4 que le procédé de concentration est réversible. L'utilisation de la dialyse permet avantageusement de conserver la concentration initiale des liposomes formant les agrégats mixtes liposomes/α-cyclodextrine de départ puisqu'il n'y a pas dilution dans la chambre de dialyse. De plus l'utilisation de la dialyse permet de garantir l'absence de molécules d'α-cyclodextrine libres dans le milieu aqueux en équilibre avec les liposomes redispersés ce qui n'exclue pas qu'une faible quantité de molécules d'α-cyclodextrine reste au final adsorbée à la surface des liposomes, donnant lieu à de nouveaux systèmes hybrides nanoparticulaires décrits comme des structures de vésicules délimitées par une bicouche de phospholipides elle-même recouverte par une ou plusieurs couches de molécules d'α-cyclodextrine.

La même expérience peut être reproduite en partant du concentré d'agrégats mixtes obtenus par le procédé de concentration puis séparé par décantation du milieu aqueux en excès. De cette façon, avantageusement, la dialyse permet de produire une dispersion de liposomes individualisés concentrés d'un facteur au moins 4 par rapport aux liposomes avant la formation des agrégats mixtes de liposomes et d'α-cyclodextrine.

La même expérience peut être reproduite en partant d'un lyophilisat des agrégats mixtes de liposomes et d'α-cyclodextrine comme décrit dans l'exemple 5 et après réhydratation de ce lyophilisat avec la quantité de milieu aqueux ajustée à la concentration finale en liposomes individualisés que l'on souhaite récupérer après l'étape de dialyse.

## Revendications

1. Procédé de préparation d'agrégats mixtes liposomes-cyclodextrines ledit procédé comprenant :
l'encapsulation d'au moins une substance exogène dans des liposomes,
la mise en contact des liposomes dispersés en milieu aqueux avec des α-cyclodextrines, lesdites α-cyclodextrines et liposomes étant présents selon un rapport des concentrations molaires cyclodextrine/liposomes dans le milieu aqueux supérieur à 1,
l'obtention d'agrégats mixtes liposomes-cyclodextrines encapsulant au moins une substance exogène.

2. Procédé de préparation d'agrégats mixtes liposomes-cyclodextrines, ledit procédé comprenant :
la mise en contact de liposomes dispersés en milieu aqueux avec des α-cyclodextrines, lesdites α-cyclodextrines et liposomes étant présents selon un rapport des concentrations molaires cyclodextrine/liposomes dans le milieu aqueux supérieur à 1,
l'obtention d'agrégats mixtes liposomes-cyclodextrines,
la lyophilisation des agrégats mixtes liposomes-cyclodextrines obtenus,
l'hydratation des agrégats mixtes liposomes-cyclodextrines lyophilisés pour obtenir des agrégats mixtes liposomes-cyclodextrines.

3. Procédé de préparation d'agrégats mixtes liposomes-cyclodextrines, ledit procédé comprenant :
la mise en contact de liposomes dispersés en milieu aqueux avec des α-cyclodextrines, lesdites α-cyclodextrines et liposomes étant présents selon un rapport des concentrations molaires cyclodextrine/liposomes dans le milieu aqueux supérieur à 1, et la concentration de liposome étant comprise entre 0.05 mM et 5mM,
l'obtention d'agrégats mixtes liposomes-cyclodextrines.

4. Procédé de préparation de liposomes, ledit procédé comprenant :
la mise en contact de liposomes dispersés en milieu aqueux avec des α-cyclodextrines, lesdites α-cyclodextrines et liposomes étant présents selon un rapport des concentrations molaires cyclodextrine/liposomes dans le milieu aqueux supérieur à 1,
l'obtention d'agrégats mixtes liposomes-cyclodextrines, et
la dialyse des d'agrégats mixtes liposomes-cyclodextrines pour obtenir les liposomes.

5. Procédé de préparation de liposomes, ledit procédé comprenant :
la mise en contact de liposomes dispersés en milieu aqueux avec des α-cyclodextrines, lesdites α-cyclodextrines et liposomes étant présents selon un rapport des concentrations molaires cyclodextrine/liposomes dans le milieu aqueux supérieur à 1,
l'obtention d'agrégats mixtes liposomes-cyclodextrines, et
la désagrégation des agrégats mixtes liposomes-cyclodextrines par dilution pour obtenir les liposomes individualisés.

6. Procédé selon l'une quelconque des revendications 1 à 5, dans lequel le rapport des concentrations molaires cyclodextrine/liposomes dans le milieu aqueux est compris entre 2 et 1500.

7. Procédé selon l'une quelconque des revendications 2 à 6, dans lequel tout ou partie des liposomes contien(nen)t une substance exogène.

8. Procédé selon la revendication 7, dans lequel la substance exogène est un principe actif cosmétique, thérapeutique ou diagnostique.

9. Procédé selon l'une quelconque des revendications 1 à 8, dans lequel les liposomes sont constitués majoritairement de phospholipides.

10. Procédé selon la revendication 7, dans lequel les phospholipides sont choisis parmi la phosphatidylcholine et ses dérivés.

11. Procédé selon l'une quelconque des revendications 1 à 8, dans lequel le milieu aqueux est tamponné à un pH compris entre 6 et 8.

12. Procédé selon l'une quelconque des revendications 1, ou 3 à 5, comprenant une étape de lyophilisation des agrégats mixtes liposomes-cyclodextrines.

13. Procédé selon l'une quelconque des revendications 1 à 3 ou 5, comprenant une étape de dialyse des agrégats mixtes liposomes-cyclodextrines.

14. Procédé selon la revendication 12, comprenant une étape de réhydratation du lyophilisat des agrégats mixtes liposomes-cyclodextrines suivie d'une étape de dialyse.

15. Lyophilisat d'agrégats mixtes liposomes-cyclodextrines susceptible d'être obtenu selon le procédé tel que défini à la revendication 12.

16. Agrégats mixtes liposomes-cyclodextrines susceptibles d'être obtenus par le procédé selon la revendication 1.

17. Agrégats mixtes liposomes-cyclodextrines selon la revendication 16, de taille comprise entre 100 nm et 10 µm.

18. Utilisation des agrégats mixtes liposomes-cyclodextrines tels que définis aux revendications 16 ou 17 pour encapsuler des substances exogènes.

19. Utilisation selon la revendication 18 pour la préparation de compositions cosmétiques, pharmaceutiques ou diagnostiques.

## Patentansprüche

1. Verfahren zur Herstellung von gemischten Liposom-Cyclodextrin-Aggregaten, wobei das Verfahren Folgendes umfasst:
die Einkapselung von wenigstens einer exogenen Substanz in Liposome,
das Inkontaktbringen der in einem wässrigen Medium dispergierten Liposome mit α-Cyclodextrinen, wobei die α-Cyclodextrine und die Liposome in einem Cyclodextrin/Liposom-Molkonzentrationsverhältnis von mehr als 1 im wässrigen Medium vorhanden sind,
das Erzielen von gemischten Liposom-Cyclodextrin-Aggregaten, die wenigstens eine exogene Substanz einkapseln.

2. Verfahren zur Herstellung von gemischten Liposom-Cyclodextrin-Aggregaten, wobei das Verfahren Folgendes umfasst:
das Inkontaktbringen von in einem wässrigen Medium dispergierten Liposomen mit α-Cyclodextrinen, wobei die α-Cyclodextrine und die Liposome in einem Cyclodextrin/Liposom-Molkonzentrationsverhältnis von mehr als 1 im wässrigen Medium vorhanden sind,
das Erzielen von gemischten Liposom-Cyclodextrin-Aggregaten,
das Lyophilisieren der erzielten gemischten Liposom-Cyclodextrin-Aggregate,
die Hydratation der lyophilisierten gemischten Liposom-Cyclodextrin-Aggregate, um gemischte Liposom-Cyclodextrin-Aggregate zu erzielen.

3. Verfahren zur Herstellung von gemischten Liposom-Cyclodextrin-Aggregaten, wobei das Verfahren Folgendes umfasst:
das Inkontaktbringen von in einem wässrigen Medium dispergierten Liposomen mit α-Cyclodextrinen, wobei die α-Cyclodextrine und die Liposome in einem Cyclodextrin/Liposom-Molkonzentrationsverhältnis von mehr als 1 im wässrigen Medium vorhanden sind und die Liposomkonzentration zwischen 0,05 mM und 5 mM beträgt,
das Erzielen von gemischten Liposom-Cyclodextrin-Aggregaten.

4. Verfahren zur Herstellung von Liposomen, wobei das Verfahren Folgendes umfasst:
das Inkontaktbringen von in einem wässrigen Medium dispergierten Liposomen mit α-Cyclodextrinen, wobei die α-Cyclodextrine und die Liposome in einem Cyclodextrin/Liposom-Molkonzentrationsverhältnis von mehr als 1 im wässrigen Medium vorhanden sind,
das Erzielen von gemischten Liposom-Cyclodextrin-Aggregaten, und
die Dialyse der gemischten Liposom-Cyclodextrin-Aggregate zur Erzielung der Liposome.

5. Verfahren zur Herstellung von Liposomen, wobei das Verfahren Folgendes umfasst:
das Inkontaktbringen von in einem wässrigen Medium dispergierten Liposomen mit α-Cyclodextrinen, wobei die α-Cyclodextrine und die Liposome in einem Cyclodextrin/Liposom-Molkonzentrationsverhältnis von mehr als 1 im wässrigen Medium vorhanden sind,
das Erzielen von gemischten Liposom-Cyclodextrin-Aggregaten, und
die Zersetzung der gemischten Liposom-Cyclodextrin-Aggregate durch Verdünnung, um einzelne Liposome zu erzielen.

6. Verfahren nach einem der Ansprüche 1 bis 5, wobei das Cyclodextrin/Liposom-Molkonzentrationsverhältnis im wässrigen Medium zwischen 2 und 1500 beträgt.

7. Verfahren nach einem der Ansprüche 2 bis 6, wobei alle oder ein Teil der Liposome eine exogene Substanz enthalten (enthält).

8. Verfahren nach Anspruch 7, wobei die exogene Substanz ein kosmetischer, therapeutischer oder diagnostischer Wirkstoff ist.

9. Verfahren nach einem der Ansprüche 1 bis 8, wobei die Liposome mehrheitlich aus Phospholipiden gebildet sind.

10. Verfahren nach Anspruch 7, wobei die Phospholipide aus Phosphatidylcholin und seinen Derivaten ausgewählt sind.

11. Verfahren nach einem der Ansprüche 1 bis 8, wobei das wässrige Medium auf einen pH zwischen 6 und 8 gepuffert ist.

12. Verfahren nach einem der Ansprüche 1 oder 3 bis 5, umfassend einen Schritt des Lyophilisierens der gemischten Liposom-Cyclodextrin-Aggregate.

13. Verfahren nach einem der Ansprüche 1 bis 3 oder 5, umfassend einen Schritt der Dialyse der gemischten Liposom-Cyclodextrin-Aggregate.

14. Verfahren nach Anspruch 12, umfassend einen Schritt der Rehydratation des Lyophilisats der gemischten Liposom-Cyclodextrin-Aggregate, gefolgt von einem Dialyseschritt.

15. Lyophilisat von gemischten Liposom-Cyclodextrin-Aggregaten, das durch das Verfahren erzielt werden kann, wie es in Anspruch 12 definiert wird.

16. Gemischte Liposom-Cyclodextrin-Aggregate, die durch das Verfahren nach Anspruch 1 erzielt werden können.

17. Gemischte Liposom-Cyclodextrin-Aggregate nach Anspruch 16 mit einer Größe zwischen 100 nm und 10 µm.

18. Verwendung der gemischten Liposom-Cyclodextrin-Aggregate, wie sie in den Ansprüchen 16 oder 17 definiert sind, zum Einkapseln von exogenen Substanzen.

19. Verwendung nach Anspruch 18 zur Herstellung von kosmetischen, pharmazeutischen oder diagnostischen Zusammensetzungen.

## Claims

1. Method for preparing mixed liposome-cyclodextrin aggregates, said method comprising:
encapsulating at least one exogenous substance in liposomes,
bringing the liposomes, which are dispersed in aqueous medium, into contact with α-cyclodextrins, said α-cyclodextrins and liposomes being present in a ratio of the cyclodextrin/liposome molar concentrations in the aqueous medium greater than 1,
obtaining mixed liposome-cyclodextrin aggregates which encapsulate at least one exogenous substance.

2. Method for preparing mixed liposome-cyclodextrin aggregates, said method comprising:
bringing liposomes, which are dispersed in aqueous medium, into contact with α-cyclodextrins, said α-cyclodextrins and liposomes being present in a ratio of the cyclodextrin/liposome molar concentrations in the aqueous medium greater than 1,
obtaining mixed liposome-cyclodextrin aggregates,
lyophilising the mixed liposome-cyclodextrin aggregates obtained,
hydrating the lyophilised mixed liposome-cyclodextrin aggregates in order to obtain mixed liposome-cyclodextrin aggregates.

3. Method for preparing mixed liposome-cyclodextrin aggregates, said method comprising:
bringing liposomes, which are dispersed in aqueous medium, into contact with α-cyclodextrins, said α-cyclodextrins and liposomes being present in a ratio of the cyclodextrin/liposome molar concentrations in the aqueous medium greater than 1, and the liposome concentration being between 0.05 mM and 5 mM,
obtaining mixed liposome-cyclodextrin aggregates.

4. Method for preparing liposomes, said method comprising:
bringing liposomes, which are dispersed in aqueous medium, into contact with α-cyclodextrins, said α-cyclodextrins and liposomes being present in a ratio of the cyclodextrin/liposome molar concentrations in the aqueous medium greater than 1,
obtaining mixed liposome-cyclodextrin aggregates, and
dialysing the mixed liposome-cyclodextrin aggregates in order to obtain liposomes.

5. Method for preparing liposomes, said method comprising:
bringing liposomes, which are dispersed in aqueous medium, into contact with α-cyclodextrins, said α-cyclodextrins and liposomes being present in a ratio of the cyclodextrin/liposome molar concentrations in the aqueous medium greater than 1,
obtaining mixed liposome-cyclodextrin aggregates, and
disaggregating the mixed liposome-cyclodextrin aggregates by dilution in order to obtain individual liposomes.

6. Method according to any one of claims 1 to 5, in which the ratio of the cyclodextrin/liposome molar concentrations in the aqueous medium is between 2 and 1500.

7. Method according to any one of claims 2 to 6, in which all or some of the liposomes contain an exogenous substance.

8. Method according to claim 7, in which the exogenous substance is a cosmetic, therapeutic or diagnostic active ingredient.

9. Method according to any one of claims 1 to 8, in which the liposomes consist mostly of phospholipids.

10. Method according to claim 7, in which the phospholipids are selected from phosphatidylcholine and derivatives thereof.

11. Method according to any one of claims 1 to 8, in which the aqueous medium is buffered to a pH between 6 and 8.

12. Method according to any one of claims 1 or 3 to 5, comprising a step of lyophilising the mixed liposome-cyclodextrin aggregates.

13. Method according to any one of claims 1 to 3 or 5, comprising a step of dialysing the mixed liposome-cyclodextrin aggregates.

14. Method according to claim 12, comprising a step of rehydrating the lyophilisate of the mixed liposome-cyclodextrin aggregates, followed by a dialysis step.

15. Lyophilisate of mixed liposome-cyclodextrin aggregates, obtainable according to the method as defined in claim 12.

16. Mixed liposome-cyclodextrin aggregates, obtainable by the method according to claim 1.

17. Mixed liposome-cyclodextrin aggregates according to claim 16, having a size between 100 nm and 10 µm.

18. Use of mixed liposome-cyclodextrin aggregates as defined in claims 16 or 17 to encapsulate exogenous substances.

19. Use according to claim 18 for the preparation of cosmetic, pharmaceutical or diagnostic compositions.
